# EUROPÄISCHE PATENTANMELDUNG

(11) **EP 4 406 566 A1**
(43) Veröffentlichungstag der Anmeldung: **31.07.2024**
(21) Anmeldenummer: 23153694.7
(22) Anmeldetag: 27.01.2023
(51) Int. Cl.: A61M 5/00, B65B 23/22, B65D 5/489, B65D 5/50, B65D 85/42, B25J 15/00

(54) **VERPACKUNG UND VERFAHREN ZUM BESTÜCKEN EINER VERPACKUNG**

(71) Anmelder: Uhlmann Pac-Systeme GmbH & Co. KG, 88471 Laupheim (DE)
(72) Erfinder: Schuster, Jochen, 88471 Laupheim (DE); Konrad, Matthias, 88471 Laupheim (DE)
(74) Vertreter: Wächter, Jochen

(57) **Zusammenfassung**

Die Verpackung (2) für Produkte (34) umfasst eine Bodenlage (4) und eine Deckellage (6), die übereinander und in einem Abstand zueinander angeordnet sind und miteinander verbunden sind, wobei die Deckellage (6) mindestens eine Aufnahmelasche (26) aufweist, die eine Mehrzahl von Ausnehmungen (32) aufweist. Die mindestens eine Aufnahmelasche (26) ist um eine Schwenkachse (A), die im Bereich der Deckellage (6) angeordnet ist, in Richtung der Bodenlage (4) schwenkbar, wobei im geschwenkten Aufnahmezustand der Aufnahmelasche (26) Eintrittsöffnungen (33) der Ausnehmungen (32) zur Einführung der Produkte (34) von der Bodenlage (4) weg zeigen und Auflageabschnitte (35) der Ausnehmungen (32) zur Stützung von Abschnitten der Produkte (34) eine den Eintrittsöffnungen (33) gegenüberliegende Begrenzung der Ausnehmungen (32) bilden.

## Beschreibung

Die vorliegende Erfindung betrifft eine Verpackung und ein Verfahren zum Bestücken einer Verpackung.

Verpackungen, insbesondere aus Karton, beispielsweise in Form von Trays, werden in vielen Industriebereichen zum Verpacken von Produkten eingesetzt. Beispiele derartiger Produkte sind auch Liquida-Produkte der pharmazeutischen Industrie, insbesondere Spritzen, Ampullen oder Vials.

Der vorliegenden Erfindung liegt die Aufgabe zugrunde, eine Verpackung anzugeben, die besonders stabil ist und in der die Produkte besonders sicher gelagert sind, sowie ein entsprechendes Verfahren zur Bestückung der Verpackung anzugeben, das auf einfache Weise erfolgen kann.

Die erfindungsgemäße Verpackung, insbesondere ein Tray, für Produkte umfasst eine Bodenlage und eine Deckellage, die übereinander und in einem Abstand zueinander angeordnet sind und miteinander verbunden sind. Die Deckellage weist mindestens eine, bevorzugt mehrere, Aufnahmelaschen auf, die jeweils eine Mehrzahl von Ausnehmungen aufweisen, wobei die mindestens eine Aufnahmelasche um mindestens eine Schwenkachse, die im Bereich der Deckellage angeordnet ist, in Richtung der Bodenlage schwenkbar ist. Im geschwenkten Aufnahmezustand der mindestens einen Aufnahmelasche zeigen Eintrittsöffnungen der Ausnehmungen zur Einführung der Produkte von der Bodenlage weg und Auflageabschnitte der Ausnehmungen zur Stützung von Abschnitten der Produkte bilden eine den Eintrittsöffnungen gegenüberliegende Begrenzung der Ausnehmungen.

Hierdurch ist die Verpackung als Ganzes stabil, die Produkte können auf einfache Weise in die Verpackung zugeführt werden und liegen sicher in den jeweiligen Ausnehmungen.

Die erfindungsgemäße Verpackung kann für viele Arten von aus Festkörpern bestehenden Produkten eingesetzt werden. Besonders geeignet ist sie insbesondere für Produkte, die eine Ausdehnung in einer Haupterstreckungsrichtung (Längsrichtung) von mindestens 20 mm, bevorzugt mindestens 30 mm, mehr bevorzugt mindestens 40 mm aufweisen. Ein spezieller Anwendungszweck liegt bei Produkten für die pharmazeutische Industrie, insbesondere bei Spritzen, Ampullen und/oder Vials.

In bevorzugter Ausgestaltung weist die Verpackung eine Zwischenbodenlage auf, die jeweils in einem Abstand zur Bodenlage und zur Deckellage zwischen der Bodenlage und der Deckellage angeordnet ist und mit der Bodenlage und/oder der Deckellage verbunden ist, wobei die Zwischenbodenlage im Schwenkbereich der mindestens einen Aufnahmelasche mindestens eine Aussparung aufweist. Hierdurch wird die Stabilität der Verpackung noch weiter erhöht.

Es ist bevorzugt, dass die mindestens eine Aussparung in der Zwischenbodenlage mindestens eine Randkontur aufweist, an der die nach unten geschwenkte Aufnahmelasche zur Anlage kommt. Auf diese Weise wird die geschwenkte Aufnahmelasche weiter stabilisiert.

In bevorzugter Ausgestaltung ist die mindestens eine Randkontur eine konvexe Kontur oder weist voneinander beabstandete Abschnitte auf, die, virtuell verbunden, eine konvexe Kontur definieren. Dadurch wird die Aufnahmelasche vorgespannt, was weiterhin zur Stabilisierung beiträgt.

Bevorzugt beträgt die Höhe der konvexen Kontur an ihrem Scheitelpunkt, gemessen ab der Schnittgerade durch die zwei Endpunkte der konvexen Kontur, zwischen 1 und 10 mm, mehr bevorzugt zwischen 3 und 5 mm.

Alternativ oder zusätzlich beträgt die Höhe der konvexen Kontur an ihrem Scheitelpunkt, gemessen ab der Schnittgerade durch die zwei Endpunkte der konvexen Kontur, vorzugsweise zwischen 1 % und 20%, mehr bevorzugt zwischen 2 und 10 %, der Breite der konvexen Kontur zwischen den zwei Endpunkten. Eine solche geringe Konvexität hat sich als optimal zur Stabilisierung herausgestellt.

Alternativ oder zusätzlich weist die konvexe Kontur vorzugsweise die Form eines Kreisausschnitts auf mit einem Krümmungsradius von zwischen 25 und 2500 mm, mehr bevorzugt zwischen 60 und 600 mm.

In bevorzugter Ausgestaltung weist die Zwischenbodenlage in der mindestens einen Aussparung und bevorzugt nahe der Randkontur mindestens eine, bevorzugt zwei symmetrisch angeordnete Einrastnasen für die nach unten geschwenkte Aufnahmelasche auf. Hierdurch wird die Stabilität der Verpackung weiter gesteigert, weil die Aufnahmelasche nur noch mit erheblicher Krafteinwirkung über die Einrastnase zurückgeschwenkt werden könnte.

Bevorzugt erstrecken sich im nicht geschwenkten Ausgangszustand die mindestens eine Aufnahmelasche und ihre Ausnehmungen im Wesentlichen plan zum restlichen Grundkörper der Deckellage. Alternativ können Sie sich auch unter einem Winkel von maximal 30°, bevorzugt maximal 20°, mehr bevorzugt maximal 10° schräg bezüglich des Grundkörpers der Deckellage erstrecken.

Im geschwenkten Zustand schneidet die Ebene, in der eine Aufnahmelasche liegt, die Bodenlage vorzugsweise unter einem Winkel von +/- 65° bis 90°, mehr bevorzugt von +/- 75° bis 85°.

Die Schwenkachse der Aufnahmelasche ist vorzugsweise durch mindestens eine Rillkante, vorzugsweise zwei symmetrisch angeordnete Rillkanten, in der Deckellage definiert. Dies vereinfacht die Herstellung der Verpackung bei sicherer Funktionalität. Es sind aber auch andere Scharnierformen denkbar.

Vorzugsweise sind die Aufnahmelasche und der Grundkörper der Deckellage jeweils an zwei gegenüberliegenden Seitenbereichen der Deckellage miteinander verbunden. Auf diese Weise wird eine sichere Schwenkung der Aufnahmelasche um zwei Befestigungspunkte erreicht.

In einer bevorzugten Ausgestaltung weist die Deckellage zwei Aufnahmelaschen auf, die gegensinnig schwenkbar sind und deren Ausnehmungen zur Aufnahme von verschiedenen Abschnitten derselben Produkte dienen. Dies dient der einfachen automatisierten Schwenkung und somit der einfachen Zuführung von langen Produkten in die Ausnehmungen.

Vorzugsweise weist die Zwischenbodenlage, versetzt zu den Aufnahmelaschen, Auflageflächen oder -linien zur Stützung weiterer Produktabschnitte und/oder Aussparungen zur Aufnahme weiterer Produktabschnitte auf.

Die Verpackung ist besonders bevorzugt vollständig aus Karton gebildet, insbesondere die Deckellage sollte aus Karton gebildet sein. Es ist aber auch denkbar, andere geeignete Materialien einzusetzen oder unterschiedliche Materialien für unterschiedliche Elemente der Verpackung zu verwenden.

Es ist auch bevorzugt, dass die gesamte Verpackung aus einem einzigen Verpackungszuschnitt gebildet wird.

Allgemein ist es bevorzugt, wenn die Verpackung eine im Wesentlichen quaderförmige Gestalt aufweist. Dabei ist die Höhe des Quaders vorzugsweise deutlich geringer, beispielsweise um mindestens den Faktor 2 oder Faktor 3, als die Länge des Quaders. Außerdem ist die Höhe des Quaders vorzugsweise deutlich geringer, beispielsweise um mindestens den Faktor 1,5 oder Faktor 2, als die Breite des Quaders.

Es sind aber auch andere Grundformen der Verpackung, vorzugsweise mit mindestens sechs Seitenflächen, denkbar. Die Verpackung kann z.B. auch eine würfelförmige Gestalt aufweisen. Grundformen mit mehr als sechs, z.B. acht oder zehn Seitenflächen, sind ebenfalls denkbar.

Grundsätzlich ist es vorteilhaft, wenn mindestens an einer Längsseite, bevorzugt an zwei Längsseiten, der Verpackung Seitenlaschen der aufgerichteten Verpackung miteinander verbunden, vorzugsweise fest verbunden, besonders bevorzugt verklebt, sind, um eine Zarge der Verpackung zu bilden. An den beiden Endseiten der Verpackung sind schließlich noch Verschlusslaschen angeordnet, deren Umklappen nach Bestückung der Verpackung für eine endseitige Schließung und eine besonders stabile Form der Verpackung sorgt.

In der Ausführungsform mit Bodenlage, Zwischenbodenlage und Deckellage ist es allgemein bevorzugt, wenn alle drei Lagen jeweils direkt oder indirekt miteinander verbunden, vorzugsweise fest verbunden, sind.

Im Falle einer Verpackung, die aus einem einzigen Verpackungszuschnitt hergestellt wird, kann beispielsweise die Bodenlage bereits an jeweils einer Seite einstückig über zwei Verbindungslaschen sowohl mit der Deckellage als auch mit der Zwischenbodenlage verbunden sein. Zwei äußere Seitenlaschen des Verpackungszuschnitts, die ursprünglich im planen Zustand des Verpackungszuschnitts an der der Bodenlage abgewandten Seite der Zwischenbodenlage bzw. der Deckellage angeordnet waren, dienen dann zur Verbindung, vorzugsweise Verklebung, mit jeweils einer der beiden Verbindungslaschen, die ursprünglich im planen Zustand des Verpackungszuschnitts zwischen Bodenlage und Zwischenbodenlage bzw. Bodenlage und Deckellage angeordnet waren. Auf diese Weise wird eine stabile Zargenform der Verpackung erzielt.

Grundsätzlich wäre es aber auch denkbar, einzelne Lagen der erfindungsgemäßen Verpackung als separate Elemente herzustellen und erst beim Zusammenbau der Verpackung miteinander zu verbinden.

Bei der erfindungsgemäßen Verpackung liegen die Produkte in den Ausnehmungen der Aufnahmelasche und gegebenenfalls zusätzlich auf den Auflageflächen der Zwischenbodenlage auf. Die Produkte liegen in der Regel derart obenauf, dass sie vom Benutzer ohne weitere Öffnungsmaßnahmen ergriffen werden können, sind also vorzugsweise unbedeckt.

Insgesamt ist es im Rahmen der Erfindung vorteilhaft, wenn die Deckellage zwar eine Vielzahl von Stegen und die mindestens eine Aufnahmelasche umfasst, große weitere Bereiche der Deckellage aber Aussparungen zeigen.

Die erfindungsgemäße Verpackung wird üblicherweise noch in eine Faltschachtel eingeschoben und gelangt in dieser Form dann in den Verkehr. Zusätzlich zu den beschriebenen Elementen der Verpackung kann aber auch noch eine zusätzliche Deckfolie auf die Stege der Deckellage der Verpackung gesiegelt werden. In einem solchen Fall könnte gegebenenfalls auch die Einführung der Verpackung in eine umgebende weitere Faltschachtel entfallen.

Die Aufnahmen der mindestens einen Aufnahmelasche können beispielsweise eine sich in Richtung der Eintrittsöffnungen verjüngende Form aufweisen. Dadurch wird gewährleistet, dass die Produkte sicher vor einem Herausfallen aus den Ausnehmungen geschützt sind. Beim Einführen der Produkte werden aufgrund der Elastizität des Materials der Aufnahmelasche diese Verjüngungen dann jeweils kurzfristig aufgebogen.

Die Aufnahmen der mindestens einen Aufnahmelasche nehmen in der Regel lediglich einen kleinen Produktabschnitt auf. Die Erstreckung der Ausnehmungen der mindestens einen Aufnahmelasche in einer Haupterstreckungsrichtung (Längsrichtung) der Produkte beträgt in der Regel maximal 5 mm, vorzugsweise maximal 3 mm, mehr bevorzugt maximal 2 mm. Die Erstreckung der Ausnehmungen der mindestens einen Aufnahmelasche senkrecht zur Haupterstreckungsrichtung der Produkte beträgt in der Regel maximal 100 mm, vorzugsweise maximal 50 mm, mehr bevorzugt maximal 10 mm.

Eine für zylindrische Produktabschnitte, wie beispielsweise von Spritzen, Ampullen und Vials, besonders geeignete Form der Ausnehmung ist eine im Wesentlichen kreisförmige Form. Der Kreisradius beträgt dann vorzugsweise maximal 100 mm, mehr bevorzugt maximal 50 mm, besonders bevorzugt maximal 10 mm.

In der Aufnahmelasche sind die jeweiligen Ausnehmungen nebeneinander sowie beabstandet zueinander angeordnet. In der Regel liegen alle Ausnehmungen einer Aufnahmelasche in derselben Ebene. Wenn mehrere Aufnahmelaschen vorliegen, liegen vorzugsweise alle Ausnehmungen aller Aufnahmelaschen in zueinander parallel verlaufenden Ebenen. Dies ist insbesondere dann der Fall, wenn unterschiedliche Produktabschnitte derselben Produkte von zwei oder mehr Aufnahmelaschen gestützt werden, wie es beispielsweise bei Spritzen der Fall sein kann. In solchen Fällen sind üblicherweise auch die Schwenkachsen der verschiedenen Aufnahmelaschen jeweils parallel zueinander angeordnet. Die verschiedenen Aufnahmelaschen drehen sich somit entweder in dieselbe Richtung oder in eine entgegengesetzte Richtung.

Es ist aber auch denkbar, das unterschiedliche Aufnahmelaschen eine unterschiedliche Ausrichtung ihrer Ausnehmungen zeigen und/oder Schwenkachsen aufweisen, die schräg oder insbesondere auch senkrecht zueinander sind.

Ein bevorzugtes Verfahren zum Bestücken der Verpackung umfasst folgende Schritte:
- Bereitstellen einer Verpackung wie oben beschrieben;
- Schwenken der mindestens einen Aufnahmelasche in Richtung der Bodenlage,
- gleichzeitiges Einführen einer Mehrzahl von Produkten in die Ausnehmungen der mindestens einen geschwenkten Aufnahmelasche.

Hierdurch wird die Bestückung auf einfache Weise realisiert.

Vorzugsweise erfolgen das Schwenken der mindestens einen Aufnahmelasche und das Einführen der Mehrzahl von Produkten in die Ausnehmungen automatisiert und in einem Vorgang.

Bevorzugt erfolgen das Schwenken der mindestens einen Aufnahmelasche und das Einführen der Mehrzahl von Produkten in die Ausnehmungen durch eine Linearbewegung einer Befüllvorrichtung in Richtung der Bodenlage. Dies dient der weiteren Vereinfachung des maschinellen Bestückungsvorgangs.

Im Rahmen der Erfindung hält die Befüllvorrichtung die Mehrzahl von Produkten beispielsweise mittels Saugern oder Greifern lösbar fest.

Beim Befüllvorgang stößt die Befüllvorrichtung vorzugsweise zunächst mit Schultern oder Vorsprüngen gegen die mindestens eine Aufnahmelasche, bevorzugt in einem Bereich nahe der Schwenkachse, wodurch der Schwenkvorgang eingeleitet wird. Bei weiterer Linearbewegung der Befüllvorrichtung in Richtung der Bodenlage tritt ein vorderer Teil der Befüllvorrichtung mit den Produkten durch die Aussparung im Grundkörper der Deckellage, welche beim Schwenken der Aufnahmelasche in vollem Umfang zugänglich wird, hindurch und der Schwenkvorgang wird bis zu einem Schwenkwinkel mit einem Betrag von vorzugsweise 65° bis 90°, mehr bevorzugt 75° bis 85° fortgesetzt. Letzterer Bereich hat sich als vorteilhaft erwiesen, weil ein geringer Abstand der Schultern oder Vorsprünge der Befüllvorrichtung von den Schwenkachsen eingehalten werden sollte, beispielsweise um gewisse Toleranzen zu tolerieren.

Am Ende des Schwenkvorgangs stößt die mindestens eine Aufnahmelasche vorzugsweise an der bevorzugt konvexen Randkontur der zugehörigen Aussparung in der Zwischenbodenlage an und wird dadurch geringfügig vorgespannt. Die konvexe Randkontur ist eine weitere Randbedingung, welche zumindest abschnittsweise einen Schwenkwinkel mit einem geringeren Betrag als 90° bedingt und zudem zu einer geringfügigen Abweichung des Schwenkwinkels in unterschiedlichen Abschnitten der Aufnahmelasche führen kann.

Wenn Einrastnasen vorhanden sind, halten diese die Aufnahmelasche zusätzlich in der geschwenkten Endposition fest. Im Falle einer konvexen Randkontur der zugehörigen Aussparung in der Zwischenbodenlage unterstützen die Einrastnasen auch die Vorspannung der Aufnahmelasche durch Verklemmung an vorzugsweise zwei gegenüberliegenden Seiten.

Das Übergeben der Produkte in die Aufnahmen erfolgt vorzugsweise ebenfalls durch Linearbewegung der Befüllvorrichtung, welche die Produkte hält, in Richtung der Bodenlage. Schwenkvorgang und Befüllvorgang müssen in der Realität nicht scharf voneinander getrennt sein, sondern können teilweise überlappend ablaufen.

Nach erfolgter Einführung der Produkte in die Ausnehmungen wird der Eingriff zwischen Befüllvorrichtung und Produkten gelöst und die dann leere Befüllvorrichtung kann durch eine Linearbewegung in Richtung von der Bodenlage weg wieder aus der Verpackung heraus bewegt werden.

Der beschriebene Vorgang stellt nur eine bevorzugte Möglichkeit der Bestückung der Verpackung dar. Es ist selbstverständlich auch möglich, zum Verschwenken der mindestens einen Aufnahmelasche und zum Einführen der Produkte in die Ausnehmungen unterschiedliche Vorrichtungen vorzusehen und/oder den Schwenkvorgang und den Befüllvorgang zu unterschiedlichen Zeitpunkten durchzuführen. Es ist selbstverständlich auch möglich, einen, mehrere oder alle Schritte manuell durchzuführen. Dann würde jedes Produkt einzeln zugeführt werden.

Neben der linearen Absenkbewegung der Befüllvorrichtung können noch andere Bewegungen erfolgen, z.B. mindestens eine weitere Linearbewegung der Befüllvorrichtung in eine andere Richtung vor dem Einführen der Produkte in die Verpackung oder ein lineares Mitbewegen der Befüllvorrichtung in Transportrichtung der Verpackung während des Befüllvorgangs im Falle eines kontinuierlichen Transports der Verpackung während des Befüllvorgangs.

Weitere Merkmale und Vorteile der vorliegenden Erfindung ergeben sich aus der nachfolgenden Beschreibung unter Bezugnahme auf die Zeichnungen.
- Fig. 1: ist eine Perspektivansicht einer Ausführungsform der erfindungsgemäßen Verpackung in einem Ausgangszustand mit nicht geschwenkten Aufnahmelaschen;
- Fig. 2: ist eine Draufsicht auf einen einstückigen Verpackungszuschnitt, aus dem die Verpackung aus Fig. 1 gebildet werden kann;
- Fig. 3: ist eine Perspektivansicht der Verpackung aus Fig. 1 in einem geschwenkten Aufnahmezustand der Aufnahmelaschen;
- Fig. 4: ist eine Perspektivansicht der Verpackung entsprechend Fig. 3, die mit Produkten gefüllt ist.
- Fig. 5: ist eine Perspektivansicht von unten einer Ausgestaltung einer Befüllvorrichtung für die Verpackung aus Fig. 1; und
- Fig. 6: ist eine vergrößerte Draufsicht auf eine Randkontur der großen Aussparung der Zwischenbodenlage aus Fig. 2.

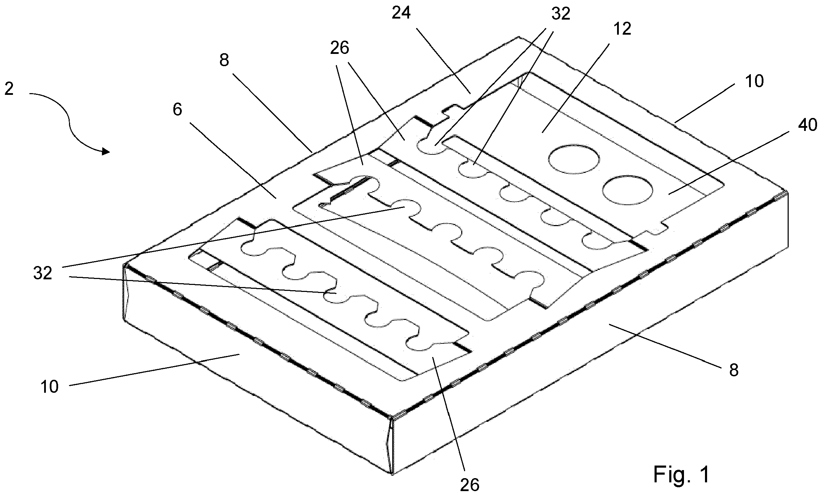

In Fig. 1 ist eine Ausführungsform der erfindungsgemäßen Verpackung 2 im Ausgangszustand dargestellt.

Die Verpackung 2 hat im Wesentlichen die Form eines Quaders mit einer Bodenlage 4, einer Deckellage 6, zwischen denen sich zwei sich gegenüberliegende Längsseiten 8 und zwei sich gegenüberliegende Endseiten 10 erstrecken.

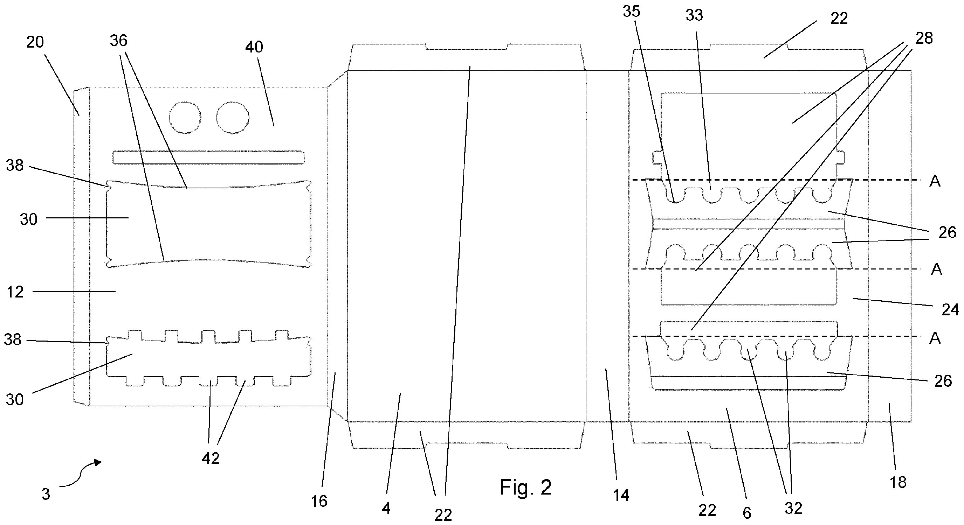

Der zugrunde liegende einstückige Verpackungszuschnitt 3 ist in Fig. 2 in Draufsicht dargestellt. Man erkennt hier neben der Bodenlage 4 und der Deckellage 6 zudem eine Zwischenbodenlage 12 der späteren Verpackung 2. Im dargestellten Beispielsfall ist die Bodenlage 4 im Verpackungszuschnitt zwischen der Deckellage 6 und der Zwischenbodenlage 12 angeordnet. Die Bodenlage 4 ist mit der Deckellage 6 mittels einer ersten Verbindungslasche 14 verbunden. Ebenso ist die Bodenlage 4 mit der Zwischenbodenlage 12 über eine zweite Verbindungslasche 16 verbunden. An der der Bodenlage 4 abgewandten Seite der Deckellage 6 ist außerdem eine erste Seitenlasche 18 angeordnet, und an der der Bodenlage 4 abgewandten Seite der Zwischenbodenlage 12 ist außerdem eine zweite Seitenlasche 20 angeordnet.

Die Verbindungslaschen 14, 16 und die Seitenlaschen 18, 20 sind jeweils schwenkbar mit den zugehörigen Lagen 4, 6, 12 verbunden. Beim Aufrichten des Verpackungszuschnitts 3 wird die Zwischenbodenlage 12 zunächst über die Bodenlage 4 nach innen geklappt, und anschließend wird die Deckellage 6 mittels der Verbindungslasche 14 über die zwei anderen Lagen 4, 12 geklappt und bildet somit die obere Begrenzung der Verpackung 2. Aufeinandertreffenden Flächen der Seitenlaschen 18, 20 und der Verbindungslaschen 14, 16 werden jeweils vorzugsweise miteinander verklebt.

Wie in Fig. 2 dargestellt, sind an der Bodenlage 4 und der Deckellage 6 zudem 2 sich jeweils gegenüberliegende Verschlusslaschen 22 angeordnet, welche nach dem Aufrichten der Verpackung 2 an den Endseiten 10 der Verpackung 2 zu liegen kommen. Die an jeweils einer Endseite 10 der Verpackung 2 sich gegenüberliegenden Verschlusslaschen 22 müssen zur Herstellung der Verpackung 2 jeweils gegensinnig verschwenkt werden und rasten dann ineinander ein. Im Ergebnis erhält man den in Fig. 1 dargestellten Zustand der Verpackung 2, im Folgenden als Ausgangszustand bezeichnet.

In der aufgerichteten Verpackung 2 liegt somit ein Sandwich aus drei Lagen vor, der Bodenlage 4, der Deckellage 6 und der dazwischenliegenden Zwischenbodenlage 12. Die drei Lagen 4, 6, 12 sind jeweils voneinander beabstandet.

Die Bodenlage 4 ist vorzugsweise aus einer durchgängigen geschlossenen Schicht gebildet.

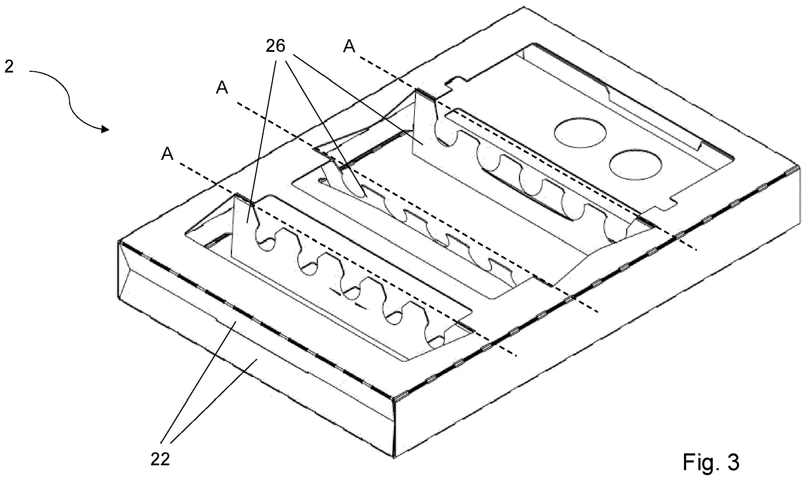

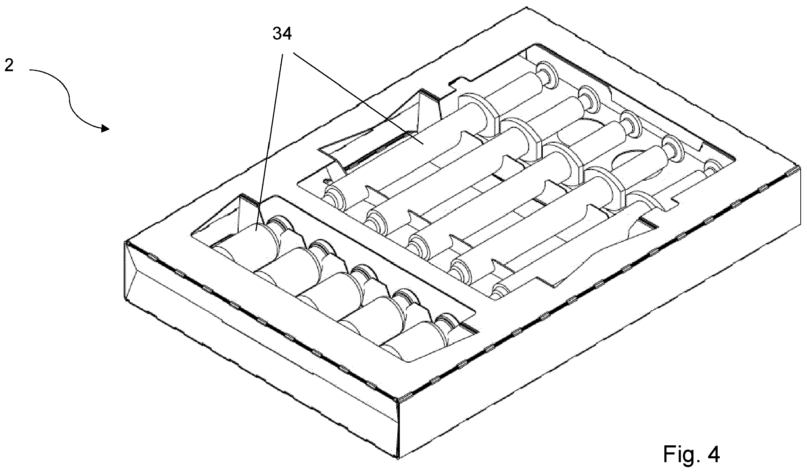

Im dargestellten Beispielsfall umfasst die Deckellage 6 einen Grundkörper 24, der aus einer Mehrzahl von zusammenhängenden Stegen gebildet ist, sowie drei Aufnahmelaschen 26. Zwischen den Stegen des Grundkörpers 24 sind zwei größere Aussparungen 28 vorgesehen, innerhalb derer sich auch die Aufnahmelaschen 26 befinden. In dem in Fig. 1 dargestellten Ausgangszustand der Verpackung 2 erstrecken sich die Aufnahmelaschen 26 im Wesentlichen plan zum Grundkörper 24 der Deckellage 6. Jede Aufnahmelasche 26 ist an zwei Randbereichen schwenkbar mit dem Grundkörper 24 verbunden. Die entsprechenden Schwenkachsen A sind in Fig. 1 eingezeichnet. Die beiden mittig angeordneten Aufnahmelaschen 26 sind hier gegensinnig schwenkbar. Jede Aufnahmelasche 26 weist eine Mehrzahl von Ausnehmungen 32 zur Stützung von Produkten 34 (siehe Fig. 4) auf, wobei im geschwenkten Aufnahmezustand (Fig. 3) der Aufnahmelasche 26 Eintrittsöffnungen 33 der Ausnehmungen 32 zur Einführung der Produkte 34 von der Bodenlage 4 weg zeigen und Auflageabschnitte 35 der Ausnehmungen 32 zur Stützung von Abschnitten der Produkte 34 eine den Eintrittsöffnungen 33 gegenüberliegende Begrenzung der Ausnehmungen 32 bilden.

Die Zwischenbodenlage 12 umfasst im dargestellten Beispielsfall zwei Aussparungen 30 im Schwenkbereich der Aufnahmelaschen 26, wie am besten aus Fig. 2 ersichtlich ist. Jede Aussparung 30 umfasst eine bzw. zwei Randkonturen 36, an denen jeweils eine nach unten geschwenkte Aufnahmelasche 26 im in Fig. 3 dargestellten geschwenkten Aufnahmezustand zur Anlage kommt. Die Randkontur 36 ist jeweils eine konvexe Kontur bzw. weist voneinander beabstandete Abschnitte auf, die, virtuell verbunden, eine konvexe Kontur definieren (Fig. 2). In jeder Aussparung 30 und nahe der Randkontur 36 weist die Zwischenbodenlage 12 außerdem zwei symmetrisch angeordnete Einrastnasen 38 für jede nach unten geschwenkte Aufnahmelasche 26 auf. Schließlich weist die Zwischenbodenlage 12, versetzt zu den Aufnahmelaschen 26, Auflageflächen oder Auflagelinien 40 zur Stützung weiterer Produktabschnitte und/oder Aussparungen 42 zur Aufnahme weiterer Produktabschnitte auf.

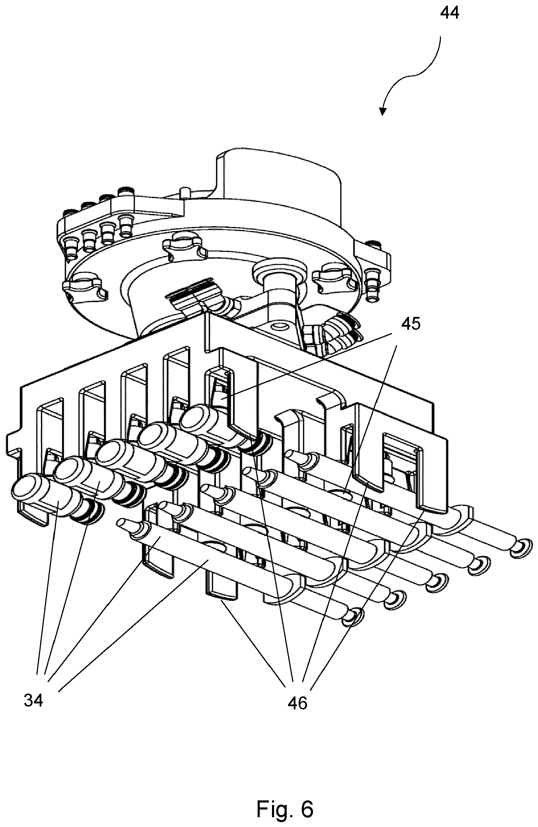

In Fig. 6 ist eine Randkontur 36 in vergrößerter Darstellung abgebildet. Bevorzugt beträgt die Höhe H der konvexen Kontur an ihrem Scheitelpunkt, gemessen ab der Schnittgerade S durch die zwei Endpunkte 43 der konvexen Kontur, zwischen 1% und 10%, mehr bevorzugt zwischen 2 und 6 %, der Breite B der konvexen Kontur zwischen den zwei Endpunkten 43. Die Höhe H der konvexen Kontur liegt beispielsweise in einem Bereich von zwischen 2 und 10 mm.

In Fig. 4 ist schließlich eine mit Produkten 34 befüllte Verpackung 2 dargestellt. Die Produkte 34 liegen hierbei in den Ausnehmungen 32 der Aufnahmelaschen 26 auf. Im dargestellten Beispielsfall sind die Produkte 34 Vials und Spritzen, wobei die Spritzen aufgrund ihrer Länge auf jeweils zwei Aufnahmelaschen 26 gelagert sind.

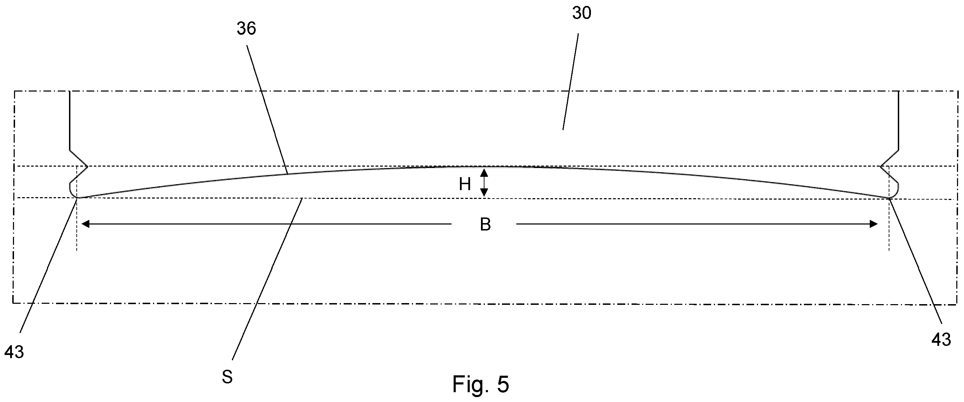

Die Befüllung der Verpackung 2 mit Produkten 34 erfolgt vorzugsweise automatisiert mittels einer Befüllvorrichtung 44 (beispielhaft in Fig. 5 dargestellt), die Sauger oder Greifer 45 für die Produkte 34 aufweist und eine Linearbewegung in Richtung der Bodenlage 4 ausführt.

Durch die Linearbewegung werden, bevorzugt mittels Schultern oder Vorsprüngen 46 der Befüllvorrichtung 44, zunächst die Aufnahmelaschen 26 in Richtung der Bodenlage 4 verschwenkt. Anschließend oder teilweise zeitlich überlappend wird durch die Linearbewegung eine Mehrzahl von Produkten 34 gleichzeitig in die Ausnehmungen 32 der geschwenkten Aufnahmelaschen 26 eingeführt.

Nach erfolgtem Befüllvorgang lässt die Befüllvorrichtung 44 die Produkte 34 los und die dann leere Befüllvorrichtung 44 kann durch eine Linearbewegung in Richtung von der Bodenlage 4 weg wieder zurückbewegt werden. Eine getaktete Befüllung ist hierbei bevorzugt. Es ist aber auch denkbar, die Befüllvorrichtung 44 mit der bewegten Verpackung 2 in einer Richtung senkrecht zur Richtung der Linearbewegung mit zu bewegen.

Neben der dargestellten Ausführungsform der Verpackung 2 sind auch viele weitere Ausführungsformen denkbar. Beispielsweise sind die Anzahl der Aufnahmelaschen 26 und deren Anordnung sowie die Schwenkrichtung beliebig variierbar. Außerdem ist es in bestimmten vereinfachten Ausführungsformen der Erfindung denkbar, die Zwischenbodenlage 12 wegzulassen.

## Patentansprüche

1. Verpackung (2) für Produkte (34) mit
einer Bodenlage (4) und einer Deckellage (6), die übereinander und in einem Abstand zueinander angeordnet sind und miteinander verbunden sind,
wobei die Deckellage (6) mindestens eine, bevorzugt mehrere, Aufnahmelaschen (26) aufweist, die jeweils eine Mehrzahl von Ausnehmungen (32) aufweisen,
wobei die mindestens eine Aufnahmelasche (26) um mindestens eine Schwenkachse (A), die im Bereich der Deckellage (6) angeordnet ist, in Richtung der Bodenlage (4) schwenkbar ist, wobei im geschwenkten Aufnahmezustand der mindestens einen Aufnahmelasche (26) Eintrittsöffnungen (33) der Ausnehmungen (32) zur Einführung der Produkte (34) von der Bodenlage (4) weg zeigen und Auflageabschnitte (35) der Ausnehmungen (32) zur Stützung von Abschnitten der Produkte (34) eine den Eintrittsöffnungen (33) gegenüberliegende Begrenzung der Ausnehmungen (32) bilden.

2. Verpackung (2) nach Anspruch 1, **dadurch gekennzeichnet, dass** sie eine Zwischenbodenlage (12) aufweist, die jeweils in einem Abstand zur Bodenlage (4) und zur Deckellage (6) zwischen der Bodenlage (4) und der Deckellage (6) angeordnet ist und mit der Bodenlage (4) und/oder der Deckellage (6) verbunden ist, wobei die Zwischenbodenlage (12) im Schwenkbereich der mindestens einen Aufnahmelasche (26) mindestens eine Aussparung (30) aufweist.

3. Verpackung (2) nach Anspruch 2, **dadurch gekennzeichnet, dass** die mindestens eine Aussparung (30) in der Zwischenbodenlage (12) mindestens eine Randkontur (36) aufweist, an der die nach unten geschwenkte Aufnahmelasche (26) zur Anlage kommt.

4. Verpackung (2) nach Anspruch 3, **dadurch gekennzeichnet, dass** die mindestens eine Randkontur (36) eine konvexe Kontur ist oder voneinander beabstandete Abschnitte aufweist, die, virtuell verbunden, eine konvexe Kontur definieren.

5. Verpackung (2) nach Anspruch 4, **dadurch gekennzeichnet, dass** die Höhe (H) der konvexen Kontur an ihrem Scheitelpunkt, gemessen ab der Schnittgerade (S) durch die zwei Endpunkte (43) der konvexen Kontur, zwischen 1 und 10 mm, bevorzugt zwischen 3 und 5 mm beträgt.

6. Verpackung (2) nach Anspruch 4 oder 5, **dadurch gekennzeichnet, dass** die konvexe Kontur die Form eines Kreisausschnitts aufweist mit einem Krümmungsradius von zwischen 25 und 2500 mm, bevorzugt zwischen 60 und 600 mm.

7. Verpackung (2) nach einem der Ansprüche 2 bis 6, **dadurch gekennzeichnet, dass** die Zwischenbodenlage (12) in der mindestens einen Aussparung (30) und bevorzugt nahe der Randkontur (36) mindestens eine, bevorzugt zwei symmetrisch angeordnete Einrastnasen (38) für die nach unten geschwenkte Aufnahmelasche (26) aufweist.

8. Verpackung (2) nach einem der vorangehenden Ansprüche, **dadurch gekennzeichnet, dass** sich im nicht geschwenkten Ausgangszustand die mindestens eine Aufnahmelasche (26) und ihre Ausnehmungen (32) im Wesentlichen plan zum restlichen Grundkörper (24) der Deckellage (6) erstrecken oder unter einem Winkel von maximal 30°, bevorzugt maximal 10°, schräg bezüglich des Grundkörpers (24) der Deckellage (6) verlaufen.

9. Verpackung (2) nach einem der vorangehenden Ansprüche, **dadurch gekennzeichnet, dass** die Schwenkachse (A) durch mindestens eine Rillkante, vorzugsweise zwei symmetrisch angeordnete Rillkanten, in der Deckellage (6) definiert ist.

10. Verpackung (2) nach einem der vorangehenden Ansprüche, **dadurch gekennzeichnet, dass** die Aufnahmelasche (26) und der Grundkörper (24) der Deckellage (6) jeweils an zwei gegenüberliegenden Seitenbereichen der Deckellage (6) miteinander verbunden sind.

11. Verpackung (2) nach einem der vorangehenden Ansprüche, **dadurch gekennzeichnet, dass** die Deckellage (6) zwei Aufnahmelaschen (26) aufweist, die gegensinnig schwenkbar sind und deren Ausnehmungen (32) zur Aufnahme von verschiedenen Abschnitten derselben Produkte (34) dienen.

12. Verpackung (2) nach einem der vorangehenden Ansprüche, **dadurch gekennzeichnet, dass** die Zwischenbodenlage (12), versetzt zu den Aufnahmelaschen (26), Auflageflächen oder Auflagelinien (40) zur Stützung weiterer Produktabschnitte aufweist und/oder Aussparungen (42) zur Aufnahme weiterer Produktabschnitte aufweist.

13. Verfahren zum Bestücken einer Verpackung (2) mit folgenden Schritten:
- Bereitstellen einer Verpackung (2) nach einem der vorangehenden Ansprüche im Ausgangszustand;
- Schwenken der mindestens einen Aufnahmelasche (26) in Richtung der Bodenlage (4);
- gleichzeitiges Einführen einer Mehrzahl von Produkten (34) in die Ausnehmungen (32) der mindestens einen geschwenkten Aufnahmelasche (26).

14. Verfahren nach Anspruch 13, **dadurch gekennzeichnet, dass** das Schwenken der mindestens einen Aufnahmelasche (26) und das Einführen der Mehrzahl von Produkten (34) in die Ausnehmungen (32) automatisiert und in einem Vorgang erfolgen.

15. Verfahren nach Anspruch 13 oder 14, **dadurch gekennzeichnet, dass** das Schwenken der mindestens einen Aufnahmelasche (26) und das Einführen der Mehrzahl von Produkten (34) in die Ausnehmungen (32) durch zumindest eine Linearbewegung einer Befüllvorrichtung (44) erfolgen.
